Europäisches Patentamt

(19) European Patent Office

Office européen des brevets

(11) Numéro de publication: **0 239 470**
**B1**

(12) **FASCICULE DE BREVET EUROPEEN**

(45) Date de publication du fascicule du brevet: 16.01.91

(21) Numéro de dépôt: 87400582.0

(22) Date de dépôt: 17.03.87

(51) Int. Cl.⁵: **C 12 P 1/00,** C 12 P 13/02, C 12 P 7/64

(54) Procédé pour l'exécution de réactions enzymatiques au sein d'un solvant organique.

(30) Priorité: 26.03.86 FR 8604352

(43) Date de publication de la demande:
30.09.87 Bulletin 87/40

(45) Mention de la délivrance du brevet:
16.01.91 Bulletin 91/03

(84) Etats contractants désignés:
AT CH DE GB LI LU NL SE

(56) Documents cités:
EP-A-0 061 023
EP-A-0 064 855
EP-A-0 118 928
EP-A-0 135 123
FR-A-2 177 051
US-A-4 166 006

ANNALS OF THE NEW YORK ACADEMY OF
SCIENCES, vol. 413, 27 décembre 1983, pages
545-547, New York, US; B. MATTIASSON et al.:
"Use of perfluorochemicals for oxygen supply to
immobilized cells"

(73) Titulaire: **SOCIETE NATIONALE ELF AQUITAINE
Tour Elf, 2, Place de la Coupole, La Défense 6
F-92400 Courbevoie (FR)**

(72) Inventeur: **Gancet, Christian
13 Lot. de la Dique Lons
F-64140 Billère (FR)**
Inventeur: **Guignard, Claude
Maison Cadillac Ossages
F-40290 Habas (FR)**
Inventeur: **Fourmentreaux, Philippe
Résidence Berlioz§Rue Berlioz
F-64000 Pau (FR)**

(74) Mandataire: **Kohn, Armand
5 Avenue Foch
F-92380 Garches (FR)**

# EP 0 239 470 B1

**Description**

La présente invention se rapporte à des réactions enzymatiques réalisées au sein de liquides organiques, c'est-à-dire à des opérations où un substrat donné, en solution ou dispersion dans un solvant organique, est soumis à l'action d'une enzyme. Elle s'applique aux différents types de réactions qui peuvent être réalisées à l'aide d'enzymes, en particulier hydrolyse, dont celle de liaisons ester, transestérifications, interestérifications, synthèses d'esters ou thioesters, amidations, etc.

Jusqu'à présent, les nombreuses réactions enzymatiques connues avaient lieu en milieu aqueux, mais lorsque le substrat à traiter est insoluble dans l'eau, comme c'est par exemple le cas des corps gras, il y aurait un intérêt évident à opérer dans un milieu capable de dissoudre ce substrat. Ainsi a-t-on proposé d'effectuer l'interestérification de matières grasses au sein de l'éther de pétrole qui les dissout (Demande de Brevet Européen 0064855), mais ce solvant est inflammable et déflagrant. Or, le choix du solvant est délicat, parce que celui-ci doit être bien compatible avec l'enzyme, dont il ne doit pas entraver l'action. Récement, on a trouvé que certains mélanges d'éthers et de cétones conviennent bien, notamment à l'hydrolyse de triglycérides (demande de brevet en France 85 06388); un progrès net est ainsi réalisé, mais on reste encore dans le domaine de matières inflammables et ces mélanges conviennent mal à d'autres réactions, notamment aux estérifications.

La présente invention est basée sur la découverte d'une classe de composés organiques qui, non seulement n'entravent pas l'action de nombreuses enzymes, mais peuvent même exalter leur action; ces composés sont ininflammable et inexplosibles et capables de dissoudre différentes subtances hydrophobes, en particulier matières grasses.

Ainsi, le procédé pour la réalisation de réactions enzymatiques au sein de solvants organiques, suivant l'invention, est-il caractérisé en ce qu'on emploie ou un plusieurs hydrocarbures fluorés ou halogéno-fluorés comprenant des halogènes autres que le fluor.

Dans la suite de la présente description, dans le but de simplification, les liquides organiques, employés suivant l'invention, pour servir de milieu réactionnel à l'enzyme, sont appelés hydrocarbures flourés, mais ce terme recouvre tant les composés seulement fluorés que ceux contiennent également d'autres halogènes, en particulier du Cl ou/et du Br.

Nombreux hydrocarbures fluorés, aliphatiques et aryliques, conviennent à la réalisation de l'invention. Cependant, comme il est préférable d'employer des composés relativement peu visqueux, convenant aux températures modérées auxquelles ont lieu les réactions enzymatiques, les dérivés fluorés préférés sont ceux des hydrocarbures en $C_1$ à $C_8$. Dans la classe des hydrocarbures fluorés aliphatiques les plus appropriés sont en $C_1$ à $C_4$, tandis que les aromatiques ont de préférence 5 à 8 C.

Parmi les hydrocarbures flourés aliphatiques, particulièrement utiles sont ceux qui correspondent à la formule générale, globale: $C_nH_mF_pX_t$ où n est 1 à 8, m = 0 à n, p étant 1 à 18 et t = 0 à 16, X étant Cl ou Br ou pouvant comprendre à la fois des atomes de Cl et Br.

Des composés fluorés bouillant au-dessous de 100°C, dont certains au-dessous de 0°C, correspondent à la même formule globale pour n = 1 à 4, m = 0 à n, p = 1 à 4 et t = 0 à 6.

Parmi les hydrocarbures fluorés, utilisable dans l'invention, se trouve le groupe connu sous la dénomination de "Fréons" ou "Foranes", comprenant des liquides frigoriques. Même ceux dont la température d'ébullition se situe au-dessous de 0°C peuvent servir à la réalisation de l'invention, à condition d'opérer sous pression, ce qui peut être parfaitement viable. Un Tableau 1 de caractéristiques de quelques uns des solvants fluorés utilisable est donné plus loin à titre d'exemples non limitatifs. On peut citer également les composés tel que; fluorodichlorométhane, fluoro-dibromométhane, fluoro-dibromo-éthane, fluoro-tétrabromométhane, fluoro-tétrachloroéthane, fluoro-tribromométhane, fluoro-trichloro-éthane, fluoro-bromodichloroéthane, difluoro-chloro-1 éthane, difluoro-dibromo-1,1 éthane, difluoro-1,2 éthane, trifluoro-dibromo-1,2 éthane, etc.

Le trichloro-fluorométhane $CFCl_3$ et le trichloro-trifluoro-éthane $CClF_2$—$CFCl_2$ méritent une mention spéciale pour leur utilité dans l'invention.

Parmi les dérivés fluorés aryliques on peut choisir par exemple parmi les fluorobenzène, fluorochloro-benzènes, fluorotoluènes, difluoro-chloro-toluènes, etc.

La plupart des composés fluorés, proposés suivant l'invention, présentent, en plus des avantages de non inflammabilité et non explosibilité, celui de l'absence de toxicité, qualité très importante pour les préparations touchant à l'alimentation, la pharmacie ou la cosmétique. On trouvera plus loin le Tableau 2 donnant le seuil des concentrations tolérables pour l'homme par voie respiratoire, exprimés en ppm et en $mg/m^3$; ce tableau compare les seuils de toxicité de 14 différents composés volatils avec ceux du trichlorotrifluoroéthane, produit type selon l'invention; il s'avère que l'organisme animal peut tolérer 100 fois plus de cet hydrocarbure chloro-fluoré que de benzène ou de chloroforme.

Suivant une variante de l'invention, l'hydrocarbure fluoré est employé en association avec un autre liquide, pour former un solvant mixte, présentant un meilleur pouvoir dissolvant vis-à-vis du substrat à traiter, une température d'ébullition convenable, une certaine capacité d'absorption de l'eau ou/et autre caractéristique physique ou physico-chimique améliorée par rapport aux composants du mélange pris séparément.

2

<u>TABLEAU 1</u>

Caractéristiques des solvants

fluorés

| Formule | "Fréons" ou "Foranes" n° | $\theta_{EB}$°C[a] | Pression de vapeur à 30°C,[b] bar |
|---|---|---|---|
| $CF_4$ | 14 | −128,0 | − |
| $CF_3Cl$ | 13 | − 81,4 | 40 |
| $CF_2Cl_2$ | 12 | − 29,8 | 7,4 |
| $CFCl_3$ | 11 | + 23,8 | 1,3 |
| $CF_2ClBr$ | 12B1 | − 4,0 | 3,3 |
| $CF_3Br$ | 13B1 | − 57,8 | 18,2 |
| $CHClF_2$ | 22 | − 40,8 | 11,9 |
| $CHF_3$ | 23 | − 82,1 | |
| $CF_3CF_3$ | 116 | − 78,2 | 40 |
| $CClF_2CF_3$ | 115 | − 38,7 | 11 |
| $CClF_2CClF_2$ | 114 | + 3,8 | 2,50 |
| $CClF_2CFCl_2$ | 113 | + 47,6 | 0,54 |
| $CCl_2FCCl_2F$ | 112 | + 92,8 | − |
| $CH_3CHF_2$ | 152 | − 24,7 | − |
| $CF_3(CF_2)_5CF_3$ | − | +101 | − |

[a]  pression absolue 1 bar

[b]  seuil de pression pour maintenir le solvant à l'état liquide.

3

**EP 0 239 470 B1**

## TABLEAU 2

### Echelle des toxicités comparées de quelques solvants (1982)

| Solvant | Seuil de concentration tolérable dans l'air | |
|---|---|---|
| | ppm | mg/m$^3$ |
| Bromure de méthyle | 5 | – |
| Tetrachlorure de carbone | 5 | 35 |
| Chloroforme | 10 | 50 |
| Benzène | 10 | 50 |
| Ammoniac | 25 | – |
| Perchloréthylène | 50 | 335 |
| Trichloréthylène | 50 | 270 |
| White Spirit | 100 | – |
| Chlorure de méthylène | 100 | 350 |
| Hexane | 100 | 360 |
| Trichloro-1,1,1 éthane | 350 | 1900 |
| Butane | 800 | – |
| Ethanol | 1000 | 1900 |
| Trichlorotrifluoroéthane | 1000 | 7600 |
| Dioxyde de carbone | 5000 | 9000 |

Ainsi peut-on avoir à utiliser un mélange d'un ou de plusieurs hydrocarbures fluorés avec des liquides comme hydrocarbures aliphatiques ou aryliques, alcools, cétones, éthers, esters, etc. Par exemple, les composés fluorés cités plus haut, ou ceux du Tableau 2, peuvent-ils être pris en mélanges avec de l'éther de pétrole, de l'hexane, de l'octane, du benzène, du toluène, du dioxane, de l'acétone, de la méthyl-éthyl cétone, de l'isopropanol, de l'acétate d'éthyle, etc., ces composés n'étant cités qu'à titre d'exemples non limitatifs. Selon la compatibilité des solvants mélangés, la proportion de composé fluoré est en général de l'ordre de 20 à 95% en poids.

Il est à noter que, grâce à l'ininflammabilité de nombreux composés fluorés, les mélanges susindiqués peuvent gagner considérablement en sécurité, même s'ils contiennent des composés inflammables à vapeurs explosives.

Bien que les composés fluorés suivant l'invention puissent être employés dans différents cas d'enzymes libres, cellesci étant dispersées dans le liquide organique, le domaine particulièrement intéressant de leurs applications est celui où l'enzyme est fixée sur un support. Cela concerne tant les systèmes d'enzymes sur support minéral, sur support minéral, sure échangeurs d'ions ou polymères, que sur tissus naturels, tels que mycellium, support cellulosique de plante ou autre.

Les hydrocarbures fluorés, appliqués selon l'invention, conviennent dans les différentes techniques enzymatiques connues, notamment en discontinu, en continu dans des colonnes ou réacteurs, sur lit fluidisé et autres.

Le domaine d'application de l'invention est très vaste, puisqu'en général les hydrocarbures fluorés peuvent être emloyés avec toutes sortes d'enzymes et substrats. Lorsque ces derniers sont hydrosolubles, cette application n s'impose pas car il est en général économique de travailler en milieu aqueux. Par contre, l'invention prend beaucoup d'intérêt dans les traitements enzymatiques de produits insolubles ou peu solubles dans l'eau; c'est le cas notamment des graisses, huiles, cires, phospholipides, notamment lécithines, stéroïdes, lipo-protéines, etc.

Ainsi, l'invention s'applique-t-elle à toutes les classes d'enzymes, protéases, oxydases, hydrolases, notamment estérases, lipases, phosphatases, transférases, etc.

Des applications industrielles fort intéressantes résident dans le traitement de corps gras par des lipases en vue de l'hydrolyse ou de la synthèse de liaisons ester, de l'interéstérification ou de la transestérification de matières grasses. Il en est de même pour des liaisons thioester ou amide.

Les conditions de température et de concentration, généralement connues dans le domaine des

4

réactions enzymatiques, s'appliquent de même à la présente invention. Ainsi, le plus souvent conviennent des températures de 10° à 80°C, et surtout 30° à 50°C. Dans le cas des estérifications d'acides gras, les concentrations préférées en acide se situent vers 0,05 à 1,5 M ou mieux de 0,1 à 1 mole par litre.

Les exemples non limitatifs, qui suivent, illustrent l'invention sur différents cas de réactions enzymatiques d'intérêt industriel. La plupart des essais ayant été effectués avec la lipase produite par le champignon *Rhizopus arrhizus* (ATCC 24 563), voici le mode de préparation de cette enzyme.

Le Rhizopus est cultivé en fermenteur dans le milieu suivant:

| | |
|---|---|
| — huile de colza | 20 g/l |
| — "Corn Steep Liquor" | 50 |
| — $KH_2PO_4$ | 2 |
| — KCl | 0,5 |
| — $NaNO_3$ | 0,5 |
| — $MgSO_4 . 7H_2O$ | 0,5 |

Le pH initial est de 4,6. La croissance a lieu pendant 3—5 jours à 25—30°C. Le mycèlium est alors essoré, lavé à l'eau distillee, et séché, soit à l'étuve sous vide à température modérée, soit par lyophilisation.

Le mycélium sec est dégraissé par extraction, puis broyé, tamisé et conservé au sec.

L'activité lipasique du mycélium ainsi préparé se situe entre 0,9 et 4 unités/mg (sur trioléine en émulsion).

Les exemples 1 à 42 montrent diverses préparations utilisant la lipase préparé comme indiqué ci-dessus, sauf l'exemple 17 portant sur l'enzyme produite par *Geotrichum Candidum* cultivé de la même façon. Les exemples 29 à 42 donnent les résultats d'une série d'essais comparatifs, montrant l'avantage des hydrocarbures fluorés sur divers autres liquides organiques, dans l'estérification d'acide gras.

Exemples 1 à 15

Esterification de l'acide oléique avec différents alcools au sein de trichlorotrifluoro-éthane

Dans chaque cas on opère sur 10 ml de solution d'un alcool et d'acide oléique, dans du $Cl_2FC—CF_2Cl$ (Fréon ou Forane 113), dans lequel on a dispersé 0,1 ou 0,2 g de mycélium dévitalisé, anhydre, de *Rhizopus arrhizus* préparé comme indiqué plus haut, ainsi que respectivement 0,1 ou 0,2 g de tamis moléculaire, pour absorber l'eau formée par l'estérification. Ainsi, selon les cas, la proportion de mycélium est de 10 ou 20 g par litre de milieu réactionnel; elle est égale à celle de tamis moléculaire. La réaction a lieu à 30°C pendant la durée indiquée pour chaque cas, au Tableau des résultats ci-après.

Le Tableau indique les concentrations en alcool et en acide oléique en moles/litre.

| Ex. n° | Alcool | Concentr.en mol/litre: | | g de mycélium par l | Durée h | Conversion % de l'acide |
|---|---|---|---|---|---|---|
| | | alcool | acide ol | | | |
| 1 | méthanol | 0,1 | 0,1 | 10 | 2,5 | 86 |
| 2 | " | 1 | 0,5 | 20 | 3 | 62 |
| 3 | éthanol | 0,1 | 0,1 | 10 | 2,5 | 91 |
| 4 | " | 1 | O,5 | 20 | 3 | 70 |
| 5 | octanol | 0,1 | 0,1 | 10 | 2,5 | 83 |
| 6 | " | 1 | 0,5 | 20 | 3 | 73 |
| 7 | butanol | 1 | 0,5 | 20 | 3 | 76 |
| 8 | alcool cinnamique | " | " | " | " | 76 |
| 9 | " iso-amylique | " | " | " | " | 83 |
| 10 | " octa-décanol | " | " | " | " | 75 |
| 11 | " hexadécanol | " | " | " | " | 79 |
| 12 | " oléique | " | " | " | " | 77 |
| 13 | décanol-1 | " | " | " | " | 78 |
| 14 | propanol-1 | " | " | " | " | 67 |
| 15 | glycérol | " | " | " | 18 | 62 |

Il apparaît que la conversion est plus élevée pour des concentrations plus faibles, 0,1 Mol/l, même après des temps un peu plus courts et avec moins de mycelium, que pour des concentrations de 1 mol/l.

Toujours est-il que le procédé de l'invention permet de réaliser des estérifications de 62 à 91% de l'acide avec divers alcools, ce qui constitue un résultat industriel très intéressant.

### Exemple 16

On opère comme dans les exemples 7 à 14 avec de l'isopropanol en tant qu'alcool et de l'acide myristique à la place de l'acide oléique. La conversion de l'acide est de 12%.

### Exemple 17

Dans l'exemple 16 le mycélium de *Rhizopus* est remplacé par celui de *Geotrichum candidum*. La conversion de l'acide gras ressort à 7,6%.

### Exemples 18 à 20

En procédant comme dans les exemples 1, 3 et 5 (concentrations 0,1 M, durée 2$^h$1/2) on a remplacé le Fréon ou Forane 113 par du Fréon ou Forane 11, c'est-à-dire du trichlorofluoro-méthane $CFCl_3$. Les conversions de l'acide oléique ont alors été;

| | |
|---|---|
| avec de l'octanol | 85,4% |
| avec du décanol | 88,3% |
| avec de l'octadécanol | 86,9% |

### Exemple 21

Esterification en continu

Le réacteur est constitué de deux colonnes contenant chacune 5,5 g d'un mycélium de *Rhizopus arrhizus* dévitalisé pour 10 ml de silice, séparées par une colonne de tamis moléculaire servant de desséchant.

Le volume mort des colonnes remplies de mycélium est de l'ordre de 15 ml pour un débit de l'ordre de 12 ml/h, ce qui correspond à un temps de séjour de 75 mn par colonne.

Le mélange réactionnel a la composition:acide oléique 0,5 M/l, octanol 0,5 M/l, solvant trichlorotri-fluoroéthane. Le taux de conversion de l'acide oléique est de 96,3% en continu.

Exemples 22 et 23

Thioestérification

Le mode opératoire est le même qu'a l'exemple 15, c'est-à-dire comme pour les exemples 6 à 14, mais durant 18 heures, les alcools étant remplacés par des mercaptans. On obtient alors les taux de conversion de l'acide oléique suivants:

| | |
|---|---|
| Ex. 22: avec du butane-thiol | 24% |
| Ex. 23: avec de l'hexane-thiol | 23%. |

Exemple 24

Transestérification

Le mélange réactionnel est constitué de 10 ml trichlorotrifluoroéthane (Fréon ou Forane 113) contenant 1 g de suif et 1 g d'acide laurique.

A ce mélange on ajoute 0,2 g de mycélium de *R. arrhizus* et on place sous agitation à 30°C pendant 20 heures.

Après séparation du mycélium, le mélange est isolé et analysé par chromatographie des dérivés tri-méthylsilyl des acides gras.

La proportion d'acides gras libérés du suif par transestérification est de 44,1%.

Exemples 25 à 27

Amidation

Le mode opératoire est de même que dans les exemples 6 à 14, plus haut, l'alcool y étant remplacé par des amines, soit 1 mol d'amine avec 0,5 mol d'acide oléique par litre; 20 g de mycélium enzymatique par litre et autant de tamis moléculaire. Le liquide flouré est le $Cl_2FC—CF_2Cl$.

Les conversions de l'acide oléique sont:

| | |
|---|---|
| Exemple 25: avec la dodécylamine | 81% |
| Exemple 26: avec l'éthanolamine | 28% |
| Exemple 27: avec l'hexaméthylène-diamine | 14% |

Exemple 28

Méthanolyse en Continu

Le réacteur est constitué par une colonne contenant 5,6 g de mycélium préparé comme précédemment et 10 ml de silice. La témpérature est de 30°C et le temps de séjour de 75 mn.

Le mélange réactionnel comprend du trichlorotrifluoroéthane (Fréon our Forane 113), contenant en poids du mélange 11,4% de suif raffiné, 2,5% de méthanol et 86,1% de ce composé flouré.

En régime continu, le taux d'acides gras librés, sous la forme d'esters méthyliques, atteint 7,9%.

Exemples 29 à 42

Estérification avec différents solvants

Aux fins de comparaison l'estérification du n-octanol par de l'acide oléique est effectuée dans le mêmes conditions, avec une série de différents solvants de cet acide gras. Les conditions sont celles des exemples 1, 3 et 5, c'est-à-dire teneurs par litre 0,1 mole pour l'acide comme pour l'alcool, 10 g de mycélium *R. arrhizus* et 10 g de tamis moléculaire; 30°C, durée 2^h30.

Voici les conversions % d'acide oléique trouvées.

| Exemple n° | Solvant | Conversion % |
|---|---|---|
| 29 | Trichloro-trifluoro-éthane (F113) | 83 |
| 30 | Trichloro-fluoro-méthane (F11) | 85,4 |
| 31 | Tétrachloro-difluoro-éthane (F112) | 85 |
| 32 | Perfluoroheptane | 81 |
| 33 | Diphényléther | 76 |
| 34 | Phtalate de dibutyle | 68 |
| 35 | Méthyl-t-butyl-éther | 56,6 |
| 36 | Diméthoxy-propane | 44,9 |
| 37 | Phosphate de tributyle | 44,2 |
| 38 | 1,2-diéthoxy-éthane | 19,4 |
| 39 | Dioxane | 11,3 |
| 40 | Méthyl-octyl cétone | 0 |
| 41 | Diméthyl-formamide | 0 |
| 42 | Diméthyl-sulfoxyde | 0 |

On peut voir que les hydrocarbures fluorés des exemples 29—32 donnent des résultats bien meilleurs que les autres solvants. Certains solvants (exemples 40—42) ne permettent pas du tout de réaliser l'estérification.

Exemple 43

Interestérification

Le mélange réactionnel est constitué de suif et d'huile de tournesol, dans le rapport pondéral 70/30 respectivement, en solution dans le trichorotrifluoroéthane 25%, v/v. Le réacteur est constitué d'une colonne contenant 5,5 g de mycelium de *Rhizopus arrhizus* dévitalisé pour 10 ml de silice, avec dessication préalable du mélange réactionnel sur tamis moléculaire.

Après un temps de séjour de 60 nm, on obtient un taux d'interéstérification de 100% pour une productivité de l'ordre de 1,5 kg de corps gras interesterifié par heure et par kg de mycélium sec, l'opération étant conduite en continu.

**Revendications**

1. Procédé pour réaliser une réaction enzymatique, le substrat traité se trouvant au sein d'un liquide organique, caractérisé en que ce liquide est un hydrocarbure fluoré pouvant renfermer des halogènes autres que le fluor.

2. Procédé suivant la revendication 1, caractérisé en ce que l'hydrocarbure fluoré est aliphatique, pouvant renfermer des atomes Cl ou/et Br.

3. Procédé suivant la revendication 2, caractérisé en ce que l'hydrocarbure fluoré répond à la composition générale $C_nH_mF_pX_t$, où n est 1 à 8, m = 0 à n, p est 1 à 18 et t = 0 à 16, X étant Cl ou Br ou pouvant comprendre à la fois des atomes de Cl et Br.

4. Procédé suivant la revendication 3, caractérisé en ce que le composé fluoro bout au-dessous de 100°C et, dans sa formule, n et 1 à 4, m = 0 à n, p = 1 à 4 et t = 0 à 6.

5. Procédé suivant une des revendications précédentes, caractérisé en ce que l'hydrocarbure fluoré est le trichlorofluoro-méthane ou le trichloro-trifluoro-éthane.

6. Procédé suivant la revendication 1, caractérisé en ce que l'hydrocarbure fluoré est arylique, en particulier en $C_5$ à $C_8$.

7. Procédé suivant une des revendications précédentes, caractérisé en ce que l'hydrocarbure fluoré est employé en mélange avec un autre liquide organique, en particulier hydrocarbure, alcool, ester, éther ou cétone, de préférence à raison de 20 à 95% en poids de composé fluoré dans le mélange.

8. Application du procédé suivant une des revendications précédentes à diverses réactions enzymatiques, utilisant en particulier des hydrolases, oxydases, phosphatases, protéases, transférases, estérases, notamment lipases, caractérisée en ce que l'enzyme est immobilisée et en suspension dans l'hydrocarbure fluoré, le substrat étant dissous ou dispersé dans ce liquide.

9. Application suivant la revendication 8, caractérisée en ce que la réaction enzymatique porte sur une matière grasse en solution dans l'hydrocarbure fluoré.

8

# EP 0 239 470 B1

**Patentansprüche**

1. Verfahren zur Durchführung einer enzymatischen Reaktion, bei dem sich das behandelte Substrat in einer organischen Flüssigkeit befindet, dadurch gekennzeichnet, dass es sich bei der Flüssigkeit um einen flourierten Kohlenwasserstoff handelt, der neben Fluor andere Halogene aufweisen kann.

2. Verfahren nach Anspruch 1, dadurch gekennzeichnet, dass der fluorierte Kohlenwasserstoff aliphatisch ist und Cl- oder/und Br-Atome aufweisen kann.

3. Verfahren nach Anspruch 2, dadurch gekennzeichnet, dass der fluorierte Kohlenwasserstoff einer Gesamtzusammensetzung $C_nH_mF_pX_t$ entspricht, wobei n 1 bis 8 ist, m 0 bis n ist, p 1 bis 18 ist und t 0 bis 16 ist, X Cl oder Br bedeutet oder gleichzeitig Cl- und Br-Atome umfassen kann.

4. Verfahren nach Anspruch 3, dadurch gekennzeichnet, dass die Fluorverbindung unter 100°C siedet und in der Formel n 1 bis 4 ist, m 0 bis n ist, p 1 bis 4 ist und t 0 bis 6 ist.

5. Verfahren nach einem der vorstehenden Ansprüche, dadurch gekennzeichnet, dass es sich beim fluorierten Kohlenwasserstoff um Trichlorfluormethan oder Trichlortrifluorethan handelt.

6. Verfahren nach Anspruch 1, dadurch gekennzeichnet, dass es sich beim fluorierten Kohlenwasserstoff um einen Arylkohlenwasserstoff, insbesondere mit 5 bis 8 Kohlenstoffatomen, handelt.

7. Verfahren nach einem der vorstehenden Ansprüche, dadurch gekennzeichnet, dass der fluorierte Kohlenwasserstoff im Gemisch mit einer anderen organischen Flüssigkeit, insbesondere einem Kohlenwasserstoff, Alkohol, Ester, Ether oder Keton, verwendet wird, vorzugsweise in einer Menge von 20 bis 95 Gew.% der fluorierten Verbindung im Gemisch, verwendet wird.

8. Anwendung des Verfahrens gemäss einen der vorstehenden Ansprüche auf verschiedene enzymatische Reaktionen, insbesondere unter Verwendung von Hydrolasen, Oxidasen, Phosphatasen, Proteasen, Transferasen, Esterasen und insbesondere Lipasen, dadurch gekennzeichnet, dass das Enzym immobilisiert ist und in Suspension im fluorierten Kohlenwasserstoff vorliegt, wobei das Substrat in dieser Flüssigkeit gelöst oder dispergiert ist.

9. Anwendung nach Anspruch 8, dadurch gekennzeichnet, dass die enzymatische Reaktion auf Fett in Lösung im fluorierten Kohlenwasserstoff ausgerichtet ist.

**Claims**

1. Process for carrying out an enzymatic reaction, the substrate treated being within an organic liquid, characterized in that the liquid is a fluorinated hydrocarbon which may contain halogens other than flourine.

2. Process according to claim 1, characterised in that the fluorinated hydrocarbon is aliphatic and may contain Cl and/or Br atoms.

3. Process according to claim 2, characterised in that the fluorinated hydrocarbon has the general composition $C_nH_mF_pX_t$ where n is 1 to 8, m = 0 to n, p is 1 to 18 and t = 0 to 16, X being Cl or Br or simultaneously Cl and Br atoms.

4. Process according to claim 3, characterised in that the fluorine compound boils below 100°C and, in its formula, n is 1 to 4, m = 0 to n, p = 1 to 4 and t = 0 to 6.

5. Process according to one of the preceding claims, characterised in that the fluorinated hydrocarbon is trichlorofluoromethane or trichlorotrifluoroethane.

6. Process according to claim 1, characterised in that the fluorinated hydrocarbon is acrylic, in particular $C_5$ to $C_8$.

7. Process according to one of the preceding claims, characterized in that the flourinated hydrocarbon is used in admixture with another organic liquid, in particular a hydrocarbon, alcohol, ester, ether or ketone, preferably in an amount of 20 to 95% by weight of fluorinated compound in the mixture.

8. Application of a process according to one of the preceding claims to various enzymatic reactions, using in particular hydrolases, oxydases, phosphateses, proteases, transferases, esterases, notably lipases, characterised in that the enzyme is immobilised and in suspension in the fluorinated hydrocarbon, the substrate being dissolved or dispersed in the liquid.

9. Application according to claim 8, characterised in that the enzymatic reaction takes place on a fatty material in solution in the fluorinated hydrocarbon.